# EUROPEAN PATENT SPECIFICATION

(11) **EP 3 528 790 B1**
(45) Date of publication and mention of the grant of the patent: **28.01.2026**
(21) Application number: 17793617.6
(22) Date of filing: 20.10.2017
(51) Int. Cl.: A61K 9/14, A61K 9/20, A61K 31/4365

(54) **PHARMACEUTICAL FORMULATIONS OF PRASUGREL AND PROCESSES FOR THE PREPARATION THEREOF**
PHARMAZEUTISCHE FORMULIERUNGEN VON PRASUGREL UND VERFAHREN ZUR HERSTELLUNG DAVON
FORMULATIONS PHARMACEUTIQUES DE PRASUGREL ET SES PROCÉDÉS DE PRÉPARATION

(30) Priority: 21.10.2016 EP 16195108
(43) Date of publication of application: 28.08.2019
(73) Proprietor: Neuraxpharm Pharmaceuticals, S.L., 08970 Sant Joan Despí (Barcelona) (ES)
(72) Inventor: DIEZ-MARTIN, Ignacio, 08970 Barcelona (ES); UBEDA-PEREZ, M.Carmen, 08970 Barcelona (ES); PABLO-ALBA, Pablo, 08970 Barcelona (ES)
(74) Representative: Hoffmann Eitle
(86) International application number: PCT/EP2017/076907
(87) International publication number: WO 2018/073437

(56) References cited:
- EP-A1- 2 564 847
- WO-A1-2010/094471

## Description

### FIELD OF INVENTION

The present invention relates to a process for the micronization of prasugrel, to prasugrel formulations for micronization, to micronized prasugrel compositions obtained or obtainable by the process of the invention and compositions comprising thereof (e.g. granulates or tablets). It further relates to the pharmaceutical use of said compositions, in particular in the treatment of coagulation disorders.

### BACKGROUND OF THE INVENTION

Prasugrel is an orally administered platelet aggregation inhibitor useful for the treatment of thrombosis and other related diseases.

EP0542411 (B1) relates to tetrahydrothienopyridine derivatives, furo and pyrrolo analogs thereof and their preparation and uses for inhibiting blood platelet aggregation, including 2-acetoxy-5- ([alpha]-cyclopropylcarbonyl-2-fluorobenzyl)-4,5,6,7-tetrahydrothieno [3,2-c]pyridine (prasugrel).

Effient^{™} 5 mg and 10 mg film-coated tablets (Eli Lilly and Company Ltd Daiichi Sankyo UK Limited) are indicated to reduce thrombotic cardiovascular events in patients with acute coronary syndrome who are to be managed with percutaneous coronary intervention (http://www.medicines.org.uk/emc/medicine/21504/SPC/Efient+5mg+&+10mg+film+coated+t ablets).

Prasugrel has low solubility in water. It is also desired to increase its chemical stability in pharmaceutical compositions by preventing in particular the oxidative and hydrolytic degradation. In addition, it is also desired that the polymorphic form remains stable in the formulation. Several formulations have previously been proposed for improving the dissolution rate of prasugrel and/or the shelf life of said compositions.

For instance, EP2100607 discloses prasugrel formulations for direct compaction into tablets which were reported to have improved storage stability, said formulation comprising prasugrel, a water-soluble polymer, a disintegrant, a diluent and a lubricant.

EP2100608 describes formulations for prasugrel improved dissolution rate wherein prasugrel is mixed while applying mechanical stress to the composition. EP2409689 reports prasugrel formulations comprising pullulan as filling agent characterized in that the weight ratio of prasugrel to pullulan is between 0.25 and 10.

WO2010/094471 discloses tablets and granulates of micronized prasugrel which are prepared by a water free process and said compositions are free of lactose or mannitol. It generically discloses that an amorphous form of prasugrel base or salts may be produced by grinding in admixture with at least one pharmaceutically acceptable additive selected from diluents, surfactants, disintegrants, stabilizers, crystallization inhibitors and/or binders, or a mixture thereof. It does not disclose however a specific formulation for micronization purposes.

US20120149727 and WO2011/098536 provide formulations where prasugrel was micronized in the presence of a water-soluble hydrophilic polymer. It is reported that said hydrophilic polymer may be selected from polyethylene glycols (e.g. PEG 6000), block copolymers of ethylene oxide and propylene oxide (e.g. poloxamers), polyoxyglycerides (e.g. Gelucire), hydroxypropylmethylcellulose, hydroxypropylcellulose, carboxymethylcellulose, hydroxyethylcellulose, polyvinylpyrrolidone, copolymers of polyvinylpyrrolidone polyoxyethylene-alkyl ethers, polymethacrylate derivatives, polyvinyl alcohol, and polyvinyl alcohol derivatives.

WO2011/098536 refers to prasugrel in micronized, crystalline form and a pharmaceutical composition comprising thereof wherein prasugrel is micronized by dry or wet milling in the presence of a hydrophilic polymer, and in the presence of SDS. The use of SDS improves the dissolution rate of a formulation of prasugrel, but this fact, increase the cost of the final pharmaceutical compositions containing it. WO2011/098536 further mentions that the weight ratio of prasugrel based on the free base to hydrophilic polymer should be > 1:4, preferably >1:1, preferably ≥ 2:1, more preferably ≥ 5:1. Preferred weight ratios of prasugrel to hydrophilic polymer were reported to be about 4:1 or about 5:1.

US20120149727, which is an application from the same applicant, relates to micronized prasugrel base in non-crystalline form and pharmaceutical compositions thereof. It describes that the active substance when processed with a water-soluble hydrophilic polymer is transformed into the non-crystalline form, for example by a melting process or by grinding. It was reported that the presence of the water-soluble hydrophilic polymer was found to stabilize the non-crystalline form of the active substance and that for this to happen it was reported to be important that sufficient polymer was present, namely the weight ratio of prasugrel based on the free base to water-soluble hydrophilic polymer should be less than 1:4, preferably ≤1:4.5, more preferably ≤1:7.5. Preferred weight ratios were mentioned to be for example about 1:5 or about 1:10. Accordingly, this application teaches away from the use of these ratios of excipient for co-grinding of prasugrel when the polymorphic form of prasugrel is to be maintained in the formulation, e.g., avoid the transformation of crystalline form into the amorphous form.

Despite some progress being made in the recent years, there is an on-going need for improved formulations of prasugrel, notably with better dissolution rate and/or chemical and polymorphic stability properties. Also, there is a need to develop an improved industrially feasible and more economical process for the preparation of pharmaceutical compositions of prasugrel with these properties.

### SUMMARY OF THE INVENTION

It has surprisingly been found that the micronization of prasugrel together with a water-insoluble hydrophilic excipient in the presence of less than 50 wt. % of a water-soluble hydrophilic polymer in the composition for micronization, preferably with a formulation which consists essentially of prasugrel and a water-insoluble hydrophilic excipient, results in a prasugrel composition with improved chemical and polymorphic stability and a good rate of dissolution.

In particular, the chemical and polymorphic stability of prasugrel was shown for the formulation of Example 2 to be improved when the excipient used during the micronization process is a water-insoluble hydrophilic excipient. This effect is shown to be particularly remarkable with respect to the co-grinding with a water-soluble hydrophilic polymer (e.g. povidone). The dissolution properties of prasugrel in a composition comprising a micronized prasugrel according to the invention were also shown to be improved.

Accordingly, the first aspect of the invention relates to a process for obtaining a micronized prasugrel composition which comprises:
a) mixing prasugrel base with a water-insoluble hydrophilic excipient, wherein the water-insoluble hydrophilic excipient is crospovidone, optionally in combination with other excipients; and
b) micronizing the composition obtained in a) wherein the weight ratio of prasugrel to water-insoluble excipient in the composition obtained in a) is 1:3.

In a second aspect, the invention relates to a micronized prasugrel composition, wherein said composition is obtained or obtainable by a process according to the first aspect.

In a third aspect, the invention refers to a prasugrel composition in the form of a tablet wherein said composition comprises:
i. the micronized prasugrel composition according to the second aspect; and
ii. a pharmaceutically acceptable excipient.

In a fourth aspect, the invention is related to a prasugrel composition according to the second or third aspect, wherein said composition is a pharmaceutical composition.

In a fifth aspect, the invention refers to a prasugrel composition according to any of the second, third or fourth aspects, for use in a method of treating coagulation disorders.

### DETAILED DESCRIPTION OF THE INVENTION

### DEFINITIONS

The term "pharmaceutically acceptable" as used herein refers to those compounds, materials, compositions, and/or dosage forms which are, within the scope of sound medical judgment, suitable for contact with the tissues of human beings and animals without excessive toxicity, irritation, allergic response, or other problem complications commensurate with a reasonable benefit/risk ratio.

As used herein, the term "substantially free" refers to a formulation containing less than 0.033%, less than 0.001%, less than 0.0005%, less than 0.0003%, or less than 0.0001% of a particular compound.

The term "treating", as used herein, unless otherwise indicated, includes the amelioration, cure, and/or maintenance of a cure (i.e., the prevention or delay of relapse) of a disease or disorder. Treatment after a disorder has started aims to reduce, alleviate, ameliorate or altogether eliminate the disorder, and/or its associated symptoms, to prevent it from becoming worse, to slow the rate of progression, or to prevent the disorder from re-occurring once it has been initially eliminated (i.e., to prevent a relapse).

The term "treatment", as used herein, unless otherwise indicated, refers to the act of "treating" defined immediately above.

The term "micronization" as used herein refers to the process of reducing the average diameter of a solid material's particles. Usually, the term micronization is used when the particles that are produced from only a few micrometres (typically less than 100 µm) to the nanometre range in diameter. Traditional micronization techniques are based on the use of friction to reduce particle size. Such methods include milling and grinding. Reduction in particle size may also take place as a result of collision and impact of the particles to each other.

The term 'jet mill' as used herein denotes a mill in which particle size is reduced by high particle acceleration produced by the expansion of micronization gas, leading to friction, collision and impact between the particles.

The term "Dₓ" as used herein means that x % of the particles in a composition (based on volume) have a diameter of equal to or below a specified D value. Thus, a D₉₀ of 10 µm means that 90 % of the particles, by volume, have a diameter of or below 10 µm. As well as using D₉₀ as a measuring reference to determine particle size, D₅₀ is sometimes used for such a purpose. Therefore, a D₅₀ of 5 µm means that 50 % of the particle population, by volume, have a diameter of equal to or below 5 µm. Also a D₁₀ of 1 µm means that 10 % of the particle population, by volume, have a diameter of equal to or below 1 µm.

As used herein, a "therapeutically effective amount" may refer to an amount of prasugrel which is effective, upon single or multiple dose administration to a subject (such as a human patient) at treating coagulation-related disorders.

The term "core" as used herein is any compressed solid material that comprises a mixture of prasugrel and pharmaceutically acceptable excipients. The term core may include single or multiple tablets, tablet cores, minitablets, capsules, granules, particles, pellets, beads, or beadlets, or any combination thereof. Preferably the core is a tablet or a tablet core. The core may be coated completely or partially with a coating. The shape and size of the cores that can be used in the invention are essentially unlimited.

The term "coating" as used herein refers to adherence, adsorption, loading and/or incorporation, to some extent, preferable uniformly, of at least one solution, dispersion or suspension coating material onto and/or into a substrate. The coating material on the substrate may be of any thickness. Preferably the coating material is a thin and uniform film applied onto the substrate. A thin and uniform film can be of the type of a "film coating" or/and an "isolating film coating" or/and an "external film coating".

### DETAILED DESCRIPTION

The first aspect of the invention relates to a process for obtaining a micronized prasugrel composition which comprises:
a) mixing prasugrel base with a water-insoluble hydrophilic excipient wherein the water-insoluble hydrophilic excipient is crospovidone; and
b) micronizing the composition obtained in a) wherein the weight ratio of prasugrel to water-insoluble hydrophilic excipient in the composition obtained in a) is 1:3;
optionally, prasugrel and the water-insoluble hydrophilic excipient may be mixed with other excipients, wherein when one of said other excipients is a water-soluble hydrophilic polymer selected from the group consisting of polyethylene glycols, block copolymers of ethylene oxide and propylene oxide, polyoxyglycerides, hydroxypropylmethylcellulose, hydroxypropylcellulose, carboxymethylcellulose, hydroxyethylcellulose, polyvinylpyrrolidone, copolymers of polyvinylpyrrolidone polyoxyethylene-alkyl ethers, polymethacrylate derivatives, polyvinyl alcohol, and polyvinyl alcohol derivatives, said water-soluble hydrophilic polymer is found in the composition obtained in a) an amount of less than 50% by weight. Unless indicated otherwise, a percentage of a compound in a composition of the present invention refers to a percentage by weight relative to the total weight, which may also be referred as w/w or wt. %.

The inventors have found that the micronization of prasugrel together with a water-insoluble hydrophilic excipient in the presence of less than 50 wt. % of a water-soluble hydrophilic polymer in the composition for micronization results in a prasugrel composition with improved chemical and polymorphic stability and a good rate of dissolution.

Prasugrel has the chemical name 2-acetoxy-5-(a-cyclopropylcarbonyl-2-fluorobenzyl)-4,5,6,7-tetrahydrothieno[3,2-c]pyridine. Prasugrel has the following structural formula:

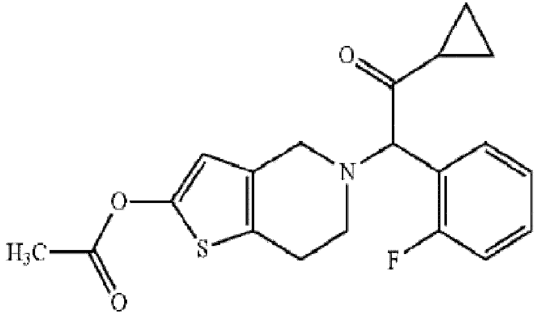

The term prasugrel as used herein refers to prasugrel base or to a pharmaceutically acceptable salt thereof.

The term "pharmaceutically acceptable salt" as used herein refers to the relatively non-toxic, inorganic and organic acid addition salts of a compound. These salts can be prepared in situ during the final isolation and purification of the compounds or by separately reacting the purified compound in its free base form with a suitable organic or inorganic acid and isolating the salt thus formed. As pharmaceutically acceptable salts of prasugrel may be mentioned for example, hydrohalides such as hydrofluoride, hydrochloride, hydrobromide or hydroiodide; inorganic acid salts such as nitrate, perchloric acid salt, sulfate or phosphate; lower-alkyl sulfonic acid salts such as methanesulfonate, trifluoromethanesulfonate or ethanesulfonate; arylsulfonic acid salts such as benzenesulfonate or p-toluenesulfonate; organic acid salts such as acetate, malate, fumarate, succinate, citrate, ascorbate, tartrate, oxalate or maleate; or amino acid salts such as glycine salt, lysine salt, arginine salt, ornithine salt, glutamic acid salt or aspartic acid salt. The preferred salts are hydrohalides or organic acid salts, more preferably hydrochloride or maleate, and most preferably hydrochloride.

Prasugrel may be in amorphous or crystalline form. Prasugrel base may be produced according to EP2985023(A1), EP0542411 or any other method known to the skilled person. For instance, amorphous prasugrel base may be prepared by precipitation from organic solvents such as alcohols, ethers, nitriles, aromates, ketones, ethers etc. Examples of such solvents are dioxane, toluene, esters, acetonitrile, tetrahydrofurane, ethyl acetate, ethanol and mixtures thereof. Also any other known crystalline form of prasugrel salts or base may be used. Methods for the preparation of crystalline prasugrel base and salts thereof are disclosed in EP1298132 and WO2011057592.

Preferably, in step a) prasugrel base is in its crystalline form.

The term "water-insoluble hydrophilic excipient" as used herein refers to water-insoluble pharmaceutically acceptable excipients for solid dosage forms for example that have a water solubility of ≤ 0.01 mg/ml at 25°C. Generally the term "water-insoluble hydrophilic excipient" designates pharmaceutically acceptable excipients with non-polar groups. According to the invention, the water-insoluble hydrophilic excipient is crospovidone.

Suitable excipients or carriers with hydrophilic properties are modified crosslinked starches, such as sodium carboxymethyl starch (sodium starch glycolate), cross-linked polyvinylpyrrolidone (crospovidone), cross-linked polymers of cellulose and derivatives (croscarmellose sodium), microcrystalline cellulose, low-substituted hydroxypropyl cellulose, ionic resins such as potassium salt of polacrilin, calcium dihydrogen phosphate, cross-linked alginic acid, and a combination thereof.

Moreover, prasugrel can be admixed in step a) with further pharmaceutically acceptable excipients if desired, provided that they do not adversely affect the desired characteristics of the formulation. The term "excipient" or "pharmaceutically acceptable excipient" refers to carriers, excipients, or vehicles such as those described herein and in Remington's Pharmaceutical Sciences 22th edition (2013).

In the event that one of said other excipients is a water-soluble hydrophilic polymer this is found in the resulting composition further to step a) in an amount of less than 50 wt. %. In one embodiment, said hydrophilic polymer is selected from the group consisting of polyethylene glycols, block copolymers of ethylene oxide and propylene oxide, polyoxyglycerides, hydroxypropylmethylcellulose, hydroxypropylcellulose, carboxymethylcellulose, hydroxyethylcellulose, polyvinylpyrrolidone, copolymers of polyvinylpyrrolidone polyoxyethylene-alkyl ethers, polymethacrylate derivatives, polyvinyl alcohol, and polyvinyl alcohol derivatives. In another embodiment, said hydrophilic polymer may be any hydrophilic polymer. The term hydrophilic polymer as used herein refers to water-soluble polymers, for example polymers that have a water solubility of >0.01 mg/ml at 25°C. Generally the designation "hydrophilic polymer" comprises polymers with polar groups. Examples of polar groups are hydroxyl, amino, carboxyl, carbonyl, ethers, esters, sulphonates. Hydroxyl groups are particularly preferred.

This water-soluble hydrophilic polymer may be found in an amount of less than 40 wt. %, 35 wt.%, 30 wt.%, 25 wt.%, 20 wt.%, or 15 wt.%, preferably of less than 10 wt.%, more preferably of less than 5 wt.%, 1 wt.%, 0.5 wt.%, 0.1 wt.%, or less than 0.05 wt.%. In a particular embodiment, the composition obtained in a) is substantially free of any of these hydrophilic polymers.

The good stability and/or dissolution rate properties of a prasugrel composition of the invention are maintained, even if a surfactant, such as ammonium lauryl sulfate or sodium dodecyl sulfate (SDS), is not used in the micronization process. Accordingly, in a preferred embodiment the composition for micronization is substantially free of a surfactant.

Preferably, the composition obtained in a) consists essentially of prasugrel and the water-insoluble hydrophilic excipient.

Prasugrel is typically used in an amount of 5 mg or 10 mg. The ratio prasugrel:water-insoluble hydrophilic excipient in the composition obtained in a) is 1:3.

Micronization in step b) can be carried out by any grinding or milling method known in the art, for example using a ball mill (planetary ball mill or mixer mill), hammer mill, rotor mill, cutting mill, bead mill, disc mill, ultrasonic mill, torus mill, impact mill, vibration mill, pin mill or air jet mill.

In a particular embodiment, the micronization step b) is characterized by one or more, preferably all, of the following features:
- the feeding rate is from 50 rpm to 300 rpm;
- the micronization speed is from 18 to 70 g/min; and
- the micronization pressure is from 1.5 to 5.5 bar.

Preferably, the micronization step is performed in the absence of water. The carrier gas used in the dry micronization process may, for example, be air, dehumidified air, dry oil-free air, noble gases, nitrogen or mixtures thereof. Air is preferred, most preferably dry oil-free air.

A preferred method and protocol for micronization is that used in the Examples. The processes of mixing and micronizing are well known in the field of pharmaceutical technology and the person skilled in the art will be able to select appropriate processing conditions.

In the micronization process it is desirable to control the particle size and particle size distribution of prasugrel. Typically, the composition obtained in a) is micronized in step b) to obtain a particle size distribution with a d10 below 10 µm, d50 below 50 µm an d90 below 100 µm. Preferably, the micronized product obtained after step b) is characterized by presenting the following particle size specifications:
- d10 between 1 and 10 µm;
- d50 between 10 and 50 µm;
- d90 between 10 and 100 µm.

More preferably, the micronized product obtained after step b) is characterized by presenting the following particle size specifications:
- d10 between 1 and 7 µm;
- d50 between 10 and 35 µm;
- d90 between 20 and 90 µm.

Particle size may be measured using different methodologies known in the art, such as by laser light diffraction or by microscopic analysis. In a particular embodiment the particle size is determined by Laser Diffraction Spectrometry (LDS), for instance with a particle size analyzer Malvern Mastersizer.

In a second aspect, the invention relates to a micronized prasugrel composition, wherein said composition is obtainable by a process according to the first aspect.

The obtained micronized prasugrel composition may be lyophilised or spray-dried. It may also be mixed with at least one additional pharmaceutical excipient preferably selected from disintegrants, diluents, and lubricants; and optionally other additives such as glidants, binders, solubility enhancers, stabilisers, antioxidant agents, emulsifiers, adsorbents, and compacted, for instance it may be granulated and/or compressed into tablets. These granulates may be used for the filling of capsules.

Different methods of compaction are known in the art, such as direct compression, roller compaction, slugging, melt extrusion or melt granulation, the direct compression method being preferred. The "direct compression method" is a method of formulation wherein the raw material powder is subjected to direct compression moulding without any pre-agglomeration processes.

Accordingly, in a third aspect, the invention refers to a prasugrel composition in the form of a tablet wherein said composition comprises:
i. the micronized prasugrel composition according to the second aspect; and
ii. a pharmaceutically acceptable excipient.

Suitable "disintegrants" may be for example, N-vinylpyrrolidone (NVP)-water-swellable polymers, crospovidone, croscarmellose sodium, carmellose sodium or calcium, cellulose derivatives such as low substituted hydroxypropyl cellulose, sodium starch glycolate, alkali and earth alkali salts of carboxymethyl cellulose and ionic resins such as potassium salt of polacrilin or other pharmaceutically acceptable disintegrants or their combinations. Preferably, a disintegrant is selected from the group consisting of croscarmellose sodium, crospovidone, and combinations thereof, preferably the disintegrant is croscarmellose sodium. The disintegrants can be added either in the process of granulating (intragranularly) and/or in the preparation of the compression mixture (extragranularly) or in both phases.

Suitable "diluents" or "fillers" may be for example, organic fillers including sugar derivatives such as lactose, sucrose, glucose, mannitol or sorbitol; starch derivatives such as corn starch, potato starch, a-starch or dextrin; cellulose derivatives such as crystalline cellulose (e.g., microcrystalline cellulose), hydroxypropylmethylcellulose, hydroxypropylcellulose, carboxymethylcellulose, preferably as sodium or calcium salt, and hydroxyethylcellulose; Arabic gum; dextran; or pullulan: or inorganic fillers including silicate derivatives such as light anhydrous silicic acid, synthetic aluminium silicate, calcium silicate or magnesium metasilicate aluminate; phosphates such as calcium hydrogenphosphate; carbonates such as calcium carbonate; or sulfates such as calcium sulfate. Of these, one or more diluents selected from cellulose derivatives and sugar derivatives are preferred. Preferably, these are selected from lactose (anhydrous or monohydrate), mannitol and microcrystalline cellulose, more preferably, the diluent or filler is selected from the list consisting of lactose monohydrate, microcrystalline cellulose and combinations thereof. These compounds may generally also be used as "binders".

Particularly, the use of lactose monohydrate in the formulation improves dosage uniformity.

The term "dosage uniformity" as used herein refers to the degree of uniformity in the amount of the active substance among dosage units; defining dosage units as dosage forms containing a single dose or a part of a dose of the active substance in each dosage unit. To ensure the dosage uniformity, each dosage unit should have an active substance content within a narrow range around the label claim. The dosage uniformity of the pharmaceutical compositions according to the present invention can be measured by means of performing the test for content uniformity, as states in European Pharmacopeia (2.9.40, monograph). As defined therein, the requirements for dosage uniformity are met if the acceptance value of the dosage units is less than or equal to 15. The acceptance value (AV) is a figure which characterized the dosage uniformity. The acceptance value is calculated, as stated in the European Pharmacopeia 7.0. (2.9.40, monograph).

Suitable "lubricants", may be for instance, stearic acid; stearic acid metal salts such as calcium stearate or magnesium stearate; sucrose stearate, talc; colloidal silica; waxes such as beeswax or spermaceti; boric acid; adipic acid; sulfates such as sodium sulfate; glycol; fumaric acid; sodium stearyl fumarate; sucrose fatty acid esters; sodium benzoate; D,L-leucine; lauryl sulfates such as sodium lauryl sulfate or magnesium lauryl sulfate; silicates such as silicic anhydride or hydrated silicate; or the aforementioned starch derivatives. Of these, sucrose stearate or fatty acid ester of sucrose is preferably used.

A sucrose stearate has shown to reduce degradation of prasugrel in accelerated stability studies for at least 3 months (40°C and 75%RH) (see "chemical and polymorphic stability results"). The sucrose fatty acid ester may be selected from any of the known sucrose fatty acid esters, for instance these may be sucrose monoesters, diesters, triesters or polyesters of lauric acid, palmitic acid and stearic acid, and combinations thereof. Preferably, the fatty acid ester is sucrose stearate. Most preferably, the fatty acid ester is sucrose stearate wherein the percentage of monoester is about 20 %.

"Glidants" may be selected from talc, colloidal silica such as Aerosil 200 or Aerosil R972 or silicates.

"Solubility enhancers" are inorganic or organic acids such as (-)-(2S,3S)-tartaric acid, (+)-(2S,3S)-di-0-benzoyltartaric acid, (+)-(2S,3S)-di-0-(4-methylbenzoyl)tartaric acid, (-)-L-phenylalanine, benzenesulfonic acid, cyclohexanesulfamic acid, naphthalene-2-sulfonic acid, sebacic acid, (+)-camphor-10-sulfonic acid, p-toluenesulfonic acid, ethanesulfonic acid, methanesulfonic acid, adipic acid, pimelic acid, maleic acid, fumaric acid, citric acid, malic acid, succinic acid, glutaric acid, malonic acid, galic acid, ferrulic acid etc. Preferably the acidic solubility enhancer is present in the pharmaceutical composition in an amount between 0.05 weight percent and 25 % w/w, preferably in an amount between 0.25 and 5 % w/w. Optionally the solubility enhancer is ground together with prasugrel.

Suitable "emulsifiers" may be for example, colloidal clays such as bentonite or bee gum; metal hydroxides such as magnesium hydroxide or aluminum hydroxide; anionic surfactants such as sodium lauryl sulfate or calcium stearate; cationic surfactants such as benzalkonium chloride; or nonionic surfactants such as polyoxyethylene alkyl ether, polyoxyethylene sorbitan fatty acid ester or sucrose fatty acid ester.

Suitable "stabilizers" may be for example, para-oxybenzoic acid esters such as methyl paraben or propyl paraben; alcohols such as chlorobutanol, benzyl alcohol or phenyl ethyl alcohol; benzalkonium chloride; phenols such as phenol orcresol; thimerosal; dehydroacetic acid; orsorbic acid and combinations thereof.

Suitable "antioxidant agents" may be for example butylated hydroxyanisole (BHA), 2,6-di-tert-butyl-4-methylphenol (BHT), propyl gallate, coffee acid, rutin quercetin, ascorbic acid or its metal salts, citric acid, edetate disodium and calcium metabisulphite, with BHA, propyl gallate and combinations thereof.

Other suitable excipients may be sweeteners, such as sodium saccharin or aspartame; acidulants, such as citric acid, malic acid or tartaric acid or flavorings.

In a particular embodiment, said prasugrel composition comprises or consists of:
- the micronized prasugrel composition obtainable by the process of the first aspect;
- a diluent, preferably lactose;
- a disintegrant; and
- optionally, a lubricant.

In a preferred embodiment, said prasugrel composition comprises or consists of:
- 7-20 wt. % of the micronized prasugrel composition obtainable by the process of the fist aspect;
- 60-80 wt. % of a diluent;
- 5-15 wt. % of a disintegrant; and
- optionally, 2-4 wt.% of a lubricant.

In the micronized prasugrel composition, prasugrel is preferably found in an amount of 2-5 wt. %, preferably about 3 wt. %, and the water-insoluble hydrophilic excipient is found in an amount of a 5-15 wt.%, preferably about 10 wt.%.

In a preferred embodiment, said prasugrel composition comprises:
- about 3 wt. % of prasugrel;
- about 70 wt. % of a diluent;
- about 10 wt. % of a disintegrant; and
- about 3 wt. % of a lubricant.

In a particular embodiment, optionally in combination with the features defined in any of the above, the pharmaceutical composition according to the third aspect of the invention, comprises or consists of:
- prasugrel;
- crospovidone;
- lactose;
- microcrystalline cellulose;
- croscarmellose sodium; and
- a sucrose fatty acid ester.

The lactose contained in the pharmaceutical composition may be anhydrous or monohydrate. Preferably, lactose used in the composition is lactose monohydrate.

In a preferred embodiment, said prasugrel composition comprises or consists of:
- 2-5 wt. % of prasugrel;
- 5-15 wt. % of crospovidone;
- 25-45 wt. % of lactose;
- 25-45 wt. % of microcrystalline cellulose;
- 5-15 wt. % of croscarmellose sodium; and
- 2-4 wt. % of a sucrose fatty acid ester.

The lactose contained in the pharmaceutical composition may be anhydrous or monohydrate. Preferably, lactose used in the composition is lactose monohydrate.

This prasugrel composition is preferably in the form of tablets. Tablets may be coated or uncoated. In addition these may be for immediate release of the active ingredient and also for sustained release. These may be obtained by different methods well known in the art, preferably by direct compression.

In a particular embodiment, the micronized prasugrel composition of the invention is mixed with the other excipients, and a lubricant is subsequently added prior to its compression into tablets.

Finally, the tablets may optionally be coated with a coating agent or composition. Conventional materials used for film coating are well known in the art and described for instance in Pharmaceutical Coating Technology (G. Cole (ed.), 1995). Film coating formulations usually contain the following components: polymer(s), plasticizer(s), colourant(s)/opacifier(s), and diluents/vehicle(s). In addition, minor quantities of flavours, surfactants and waxes may also be used.

The majority of the "polymers" used in film coating are either cellulose derivatives, such as cellulose ethers, such as hydroxyethylcellulose, hydroxypropylcellulose, hydroxypropylmethylcellulose (hypromellose) and methylcellulose, or acrylic polymers and copolymers. Occasionally encountered are high molecular weight polyethylene glycols, polyvinyl pyrrolidone, polyvinyl alcohol and waxy materials. Their function usually is to prevent bad mouth feel and/or taste and in some cases degradation, e.g. oxidation of the active ingredients and/or pharmaceutical additives used.

Commonly used "plasticizers" can be categorized into three groups:
- polyols such as glycerol, propylene glycol and macrogols,
- organic esters such as phthalate esters, dibutyl sebacetate, citrate esters and triacetin,
- oils/glycerides such as castor oil, acetylated monoglycerides and fractionated coconut oil.

The coloring agent/opacifier may be for instance water insoluble pigments such as titanium oxide, iron oxide, iron sesquioxide, yellow iron sesquioxide or water soluble colourants.

In a particular embodiment, a composition of coating suspension (calculated on dry material) comprises or consists of:
- 1-99 wt. %, preferably 1-95 wt. %, of a suitable polymer for coating applications,
- 1-50 wt. %, preferably 1-40 wt. %, of a plasticizer,
- 20 wt. %, preferably 0.1-10 wt. %, of a colourant/opacifier.

In a preferred embodiment, said coating composition comprises or consists of: hypromellose (polymer), lactose monohydrate as a diluent, titanium dioxide as a colorant/opacifier, triacetine as a plasticizer, talc as a colorant/opacifier, yellow iron oxide and optionally red iron oxide as a colorant/opacifier. Preferably said coating agent is the commercially available coating agent Opadry II (Colorcon).

In a fourth aspect, the invention is related to a prasugrel composition according to the second or third aspect, wherein said composition is a pharmaceutical composition and the use thereof as a medicament.

In a fifth aspect, the invention refers to a prasugrel composition according to any of the second, third or fourth aspects, for use in a method of treating coagulation disorders. A related aspect, is directed to a method of treating coagulation disorders with a composition or pharmaceutical composition of the present invention. In addition, the present invention provides a method of treating coagulation disorders comprising administering to a subject in need of such treatment a prophylactic or therapeutically effective amount of the pharmaceutical composition of the invention. In a preferred embodiment of any of the above, said method of treating coagulation disorders is a method for the prevention of atherothrombotic events in adult patients with acute coronary syndrome.

It is contemplated that any features described herein can optionally be combined with any of the embodiments of any composition, pharmaceutical composition, medical use, method of treatment, or process for the manufacturing of a pharmaceutical composition of the invention; and any embodiment discussed in this specification can be implemented with respect to any of these. It will be understood that particular embodiments described herein are shown by way of illustration and not as limitations of the invention. The principal features of this invention can be employed in various embodiments without departing from the scope of the invention.

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of" excludes any element, step, or ingredient not specified in the claim except for, e.g., impurities ordinarily associated with the element or limitation.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, 10, 11, 12, 13, 14 or 15%. Accordingly, the term "about" may mean the indicated value ± 5% of its value, preferably the indicated value ± 2% of its value, most preferably the term "about" means exactly the indicated value (± 0%).

The following examples serve to illustrate the present invention and should not be construed as limiting the scope thereof.

### EXAMPLES

### General Methods

**Laser Diffraction Spectrometry (LDS)** was used to determine the Particle Size Distribution (PSD). The samples were prepared by mixing 200 mg of sample with 5 mL of water containing 0.1% of Tween 80. Ultrasonic treatment of samples was performed for 5 minutes and the PSD was analysed using a particle size analyzer Malvern Mastersizer 3000.

**Powder X-Ray Diffraction (PXRD) analysis:** Approximately 15 mg of non-manipulated sample were prepared in standard sample holders using two foils of polyacetate. Powder diffraction patterns were acquired on a D8 Advance Series 2-Theta/Theta powder diffraction system using CuK_{α1}-radiation in transmission geometry. The system is equipped with a VÅNTEC-1 single photon counting PSD, a Germanium monochromator, a ninety positions auto changer sample stage, fixed divergence slits and a radial soller. Programs used: Data collection with DIFFRAC plus XRD Commander V.2.5.1, and evaluation and area integration with EVA V.14.0.0.0 (Bruker-AXS 1996-2007). The samples were measured in a range from 4° to 40° in 2 theta in 10 minutes or 1 hour measurements.

### Example 1: Co-grinding of Prasugrel with an excipient

100 g of prasugrel base was mixed with 300 g of crospovidone and the mixture was homogenized in a blender for 30 min. Then the homogenized mixture was micronized at room temperature (15-30°C) in an MICRO-MACINAZIONE Model MC-50 KX at a micronization pressure of 1,5 bar, at a feed rate of 250 rpm and a micronization speed from 18-70 g/min obtaining a mixture having a particle size distribution of d10 2.3 µm, d50 13.2 µm and d90 29.8 µm.

### Example 2: Tablet formulation

The micronized product obtained in Example 1 was mixed in a blender with Lactose monohydrate, sodium croscarmellose microcrystalline cellulose, sucrose fatty acid ester in the quantities described in table 1. The obtained powder blend was directly compressed into tablets. The ratio of prasugrel to crospovidone in the tablet is of 1:3.

**Table 1: Quantitative composition of 5 mg and 10 mg prasugrel tablets**

| **Component** | **Formulation 1** Prasugrel 5 mg | **Formulation 2** Prasugrel 10 mg |
|---|---|---|
| | **Quantity of components in mg per/ tablet** | **Quantity of components in mq/tablet** |
| Prasugrel (co-micronised example 1) | 5.00 | 10.00 |
| Crospovidone (co-micronised example 1) | 15.00 | 30.00 |
| Lactose monohydrate | 55.69 | 111.37 |
| Sodium Croscarmellose | 15.00 | 30.00 |
| Microcrystalline cellulose | 54.82 | 109.63 |
| Sucrose fatty acid ester | 4.50 | 9.00 |
| Total weight of the tablet in mg | 150.01 | 310 |

### Example 3: Co-grinding of Prasugrel with an excipient (not according to the invention)

100 g of prasugrel base was mixed with 500 g of crospovidone and the mixture was homogenized in a blender for 30 min. Then the homogenized mixture was micronized at room temperature (15-30°C) in an MICRO-MACINAZIONE Model MC-50 KX at a micronization pressure of 1,5 bar at a feed rate of 250 rpm and a micronization speed from 18-70 g/min obtaining a mixture having a particle size distribution of d10 2.6 µm, d50 14.8 µm and d90 30.8 µm.

### Example 4: Tablet formulation (not according to the invention)

The micronized product obtained in Example 3 was mixed in a blender with Lactose monohydrate, sodium croscarmellose, microcrystalline cellulose, sucrose fatty acid ester in the quantities described in table 2. The obtained powder blend was directly compressed into tablets. The ratio of prasugrel to crospovidone is of 1:5.

**Table 2: Quantitative composition of 10 mg prasugrel tablets**

| **Component** | **Formulation 3 Quantity of components in mg per tablet** |
|---|---|
| Prasugrel:crospovidone (co-micronised of example 3) | 69,60 |
| Lactose monohydrate | 95,00 |
| Croscarmellose, sodium | 30,00 |
| Cellulose, microcrystalline | 90,40 |
| Sucrose fatty acid ester | 9,00 |
| Total weight of the tablet in mg | 294 |

### Example 5: Co-grinding with an excipient (not according to the invention)

100 g of prasugrel base was mixed with 600 g of crospovidone and the mixture was homogenized for 30 min. Then the homogenized mixture was micronized at room temperature (15-30°C) in an Ultra Centrifugal Mill ZM 200, coupled with a 0.2 mm trapezoid hole sieve at a feed rate of 6000 rpm and obtaining a particle size distribution of D₁₀ 1.2 µm, D₅₀ 10 µm and D₉₀ 37 µm. The ratio of prasugrel to povidone is of 1:6.

### Example 6: Tablet formulation (not according to the invention)

The obtained micronized product of example 5 was mixed with the other excipients prior to compression into tablets. The tablets are compressed directly from powder blends of the active ingredient and the excipients (including diluents, fillers, disintegrants and lubricants), which flow uniformly into a die cavity and form a firm compact.

**Table 3: Quantitative composition of formulation 4.**

| **Component** | **Formulation 4 Quantity of components in mg per tablet** |
|---|---|
| Prasugrel:crospovidone (co-micronised example 5) | 81,68 |
| Lactose monohydrate | 77,52 |
| Cellulose, microcrystalline | 100,00 |
| Sucrose fatty acid ester | 3,90 |
| Glycerol behenate | 3,90 |
| Total weight of the tablet in mg | 267 |

### Example 7: Tablet formulation (not according to the invention)

The obtained micronized product of example 5 was mixed with the other excipients prior to compression into tablets. The tablets are compressed directly from powder blends of the active ingredient and the excipients (including diluents, fillers, disintegrants and lubricants), which flow uniformly into a die cavity and form a firm compact.

**Table 4: Quantitative composition of formulation 4a.**

| **Component** | **Formulation 4a Quantity of components in mg per tablet** |
|---|---|
| Prasugrel:crospovidone (co-micronised example 5) | 81,68 |
| Lactose monohydrate | 82,32 |
| Cellulose, microcrystalline | 100,00 |
| Magnesium stearate | 3,00 |
| Total weight of the tablet in mg | 267 |

### Comparative example 1: Co-grinding of prasugrel with a water soluble excipient

100 g of prasugrel base was mixed with 500 g of povidone and the mixture was homogenized in a blender for 30 min. Then the homogenized mixture was micronized at room temperature (15-30°C) in an MICRO-MACINAZIONE Model MC-50 KX at a micronization pressure of 1,5 bar at a feed rate of 250 rpm and a micronization speed from 18-70 g/min. obtaining a particle size distribution of d10 6.2 µm, d50 39.2 µm and d90 100.3 µm. The ratio of prasugrel to povidone is of 1:5.

### Comparative example 2: Tablet formulation

The obtained micronized product of comparative example 1 was mixed with the other excipients prior to compression into tablets. The tablets are compressed directly from powder blends of the active ingredient and the excipients (including diluents, fillers, disintegrants and lubricants), which flow uniformly into a die cavity and form a firm compact.

**Table 5: Quantitative composition of formulation 5**

| **Component** | **Formulation 5 Quantity of components in mg per tablet** |
|---|---|
| Prasugrel:Povidone (co-micronised of comparative example 1) | 63,42 |
| Lactose monohydrate | 95,00 |
| Croscarmellose, sodium | 30,00 |
| Cellulose, microcrystalline | 96,58 |
| Sucrose fatty acid ester | 9,00 |
| Total weight of the tablet in mg | 294 |

### Comparative example 3: Co-grinding of prasugrel with a water soluble excipient

100 g of prasugrel base was mixed with 500 g of mannitol and the mixture was homogenized in a blender for 30 min. Then the homogenized mixture was micronized at room temperature (15-30°C) in an MICRO-MACINAZIONE Model MC-50 KX at a micronization pressure of 1,5 bar at a feed rate of 250 rpm and a micronization speed from 18-70 g/min.

### Comparative example 4: Tablet formulation

The obtained co-micronized product of comparative example 3 was mixed in a blender with the other excipients prior to compression into tablets. The tablets are compressed directly from powder blends of the active ingredient and the excipients (including diluents, fillers, disintegrants and lubricants), which flow uniformly into a die cavity and form a firm compact.

**Table 6: Quantitative composition of formulation 6**

| **Component** | **Formulation 6 Quantity of components in mg per tablet** |
|---|---|
| Prasugrel:mannitol (co-micronised of comparative example 3) | 66,82 |
| Lactose monohydrate | 95,00 |
| Croscarmellose, sodium | 30,00 |
| Cellulose, microcrystalline | 93,18 |
| Sucrose fatty acid ester | 9,00 |
| Total weight of the tablet in mg | 294 |

### Chemical and polymorphic stability results

The formulation 2 of example 2 was stored for six months under conditions of 25 °C and 60 % relative humid (RH). The results of the stability studies are shown below, demonstrate a minimum increase in the amount of total impurities is generated over long storage periods of 3 and 6 months. In addition, the polymorphic form of prasugrel was maintained. The polymorphic stability was measured by DSC and PRXD methods.

**Table 7: Chemical and polymorphic stability of formulation 2.**

| **Total impurities %** | | | **Polymorphic form** |
|---|---|---|---|
| Initially | 3 month 25°C/60% RH | 6 month 25°C/60% RH | 6 month 25°C/60% RH |
| 0,3 | 0,4 | 0,4 | The polymorphic form was maintained |

The formulation 3, formulation 5 and formulation 6 were stored for three months under accelerated conditions at 40 °C and 75 % relative humid (RH). The stability results shown below, demonstrate a very low content of total impurities over a period of up to 3 months.

**Table 8: Chemical stability of formulation 3, formulation 5 and formulation 6.**

| | Total impurities % | | |
|---|---|---|---|
| | Initially (0 months) | 1 month 40°C/75% RH | 3 month 40°C/75% RH |
| Formulation 3 | 0,4 | 0,6 | 0,7 |
| Formulation 5 | 0,1 | 0,8 | 1,0 |
| Formulation 6 | 0,1 | 0,8 | 1,0 |

As demonstrated in the table above, the chemical stability of the micronized compositions of the third aspect is improved when prasugrel is micronized in the presence of a water-insoluble hydrophilic excipient, crospovidone (formulation 3). The degradation of formulation 3 that is prepared according to the first aspect of the invention is significantly less than the degradation of formulation 5 and 6.

### Dissolution rate studies

**Table 9: Dissolution rate of formulation 3, formulation 5 and formulation 6.**

| | **Release profile 15 min** | **Release profile 30 min** |
|---|---|---|
| Formulation 3 | 100 | 103 |
| Formulation 5 | 92 | 101 |
| Formulation 6 | 90 | 100 |

As demonstrated in the table 9 above, the dissolution rate of the micronized compositions of the third aspect is improved when prasugrel is micronized in the presence of a water-insoluble hydrophilic excipient, crospovidone (formulation 3).

## Claims

1. Process for obtaining a micronized prasugrel base composition which comprises:
a) mixing prasugrel base with a water-insoluble hydrophilic excipient wherein the water-insoluble hydrophilic excipient is crospovidone, optionally in combination with other excipients; and
b) micronizing the composition obtained in a) wherein the weight ratio of prasugrel to water-insoluble hydrophilic excipient in the composition obtained in a) is 1:3.

2. The process according to claim 1, wherein the composition obtained in a) consists essentially of prasugrel and a water-insoluble hydrophilic excipient.

3. The process according to any of claims 1 to 2, wherein said prasugrel base in step a) is prasugrel base in crystalline form.

4. The process according to any of claims 1 to 3, wherein the composition obtained in a) is micronized in step b) to an average particle size below 90 µm, preferably between 10 and 50 µm, when the average particle size is determined by Laser Diffraction Spectrometry.

5. The process according to any of claims 1 to 4, wherein the micronization step b) is **characterized by** one or more, preferably all, of the following features:
- the feeding rate is from 50 rpm to 300 rpm;
- the micronization speed is from 18 to 70 g/min; and
- the micronization pressure is from 1.5 to 5.5 bar.

6. The process according to any of claims 1 to 5, wherein said micronization step is performed by dry grinding.

7. A micronized prasugrel composition, wherein said composition is obtainable by a process according to any of claims 1 to 6.

8. A prasugrel composition in the form of a tablet wherein said composition comprises:
i. the micronized prasugrel composition according to claim 7; and
ii. a pharmaceutically acceptable excipient.

9. The prasugrel composition according to claim 8, wherein said composition comprises:
- the micronized prasugrel composition according to claim 7;
- a diluent;
- a disintegrant; and
- optionally, a lubricant.

10. The prasugrel composition according to any of claims 8 or 9, wherein said composition does not contain a surfactant.

11. The prasugrel composition according to any of claims 8 to 10, wherein said composition comprises:
- 7-20 wt. % of the micronized prasugrel composition according to claim 7;
- 60-80 wt. % of a diluent selected from the group consisting of lactose, microcrystalline cellulose and combinations thereof;
- 5-15 wt. % of a disintegrant selected from the group consisting of crospovidone, croscarmellose sodium, low-substituted hydroxypropyl cellulose, sodium starch glycolate, ionic resins such as potassium salt of polacrilin and combinations thereof, preferably is croscarmellose sodium; and
- optionally, 2-4 wt. % of a lubricant, preferably the lubricant is a sucrose fatty acid ester.

12. The prasugrel composition according to claim 11, wherein said composition comprises sucrose fatty acid ester as a lubricant and said sucrose fatty acid ester is selected from the group consisting of sucrose monoesters of lauric acid, palmitic acid and stearic acid, and combinations thereof.

13. The prasugrel composition according to any of claims 8 to 12, wherein said composition is in the form of coated tablets.

14. The prasugrel composition according to any of claims 8 to 13, for use in a method of treating coagulation disorders.

## Patentansprüche

1. Verfahren zum Erhalten einer mikronisierten Prasugrelbasiszusammensetzung, welche Folgendes umfasst:
a) das Mischen der Prasugrelbasis mit einem wasserunlöslichen hydrophilen Hilfsstoff, wobei der wasserunlösliche hydrophile Hilfsstoff Crospovidon ist, wahlweise in Kombination mit anderen Hilfsstoffen; und
b) das Mikronisieren der in a) erhaltenen Zusammensetzung, wobei das Gewichtsverhältnis von Prasugrel zum wasserunlöslichen hydrophilen Hilfsstoff in der in a) erhaltenen Zusammensetzung 1:3 ist.

2. Verfahren nach Anspruch 1, wobei die in a) erhaltenen Zusammensetzung im Wesentlichen aus Prasugrel und einem wasserunlöslichen hydrophilen Hilfsstoff besteht.

3. Verfahren nach einem der Ansprüche 1 bis 2, wobei die genannte Prasugrelbasis in Schritt a) Prasugrelbasis in kristalliner Form ist.

4. Verfahren nach einem der Ansprüche 1 bis 3, wobei die in a) erhaltenen Zusammensetzung in Schritt b) zu einer durchschnittlichen Partikelgröße unter 90 µm, vorzugsweise zwischen 10 und 50 µm, mikronisiert wird, wenn die durchschnittliche Partikelgröße mittels Laserbeugungsspektrometrie bestimmt wird.

5. Verfahren nach einem der Ansprüche 1 bis 4, wobei der Mikronisierungsschritt b) durch ein oder mehrere, vorzugsweise alle, der folgenden Merkmale gekennzeichnet ist:
- die Einspeiserate von 50 UPM bis 300 UPM ist;
- die Mikronisierungsgeschwindigkeit von 18 bis 70 g/min ist; und
- der Mikronisierungsdruck von 1,5 bis 5,5 bar ist.

6. Verfahren nach einem der Ansprüche 1 bis 5, wobei der genannte Mikronisierungsschritt mittels Trockenmahlung durchgeführt wird.

7. Mikronisierte Prasugrelzusammensetzung, wobei die genannte Zusammensetzung mittels eines Verfahrens nach einem der Ansprüche 1 bis 6 erhalten werden kann.

8. Prasugrelzusammensetzung in Form einer Tablette, wobei die genannte Zusammensetzung Folgendes umfasst:
i. die mikronisierte Prasugrelzusammensetzung nach Anspruch 7; und
ii. einen pharmazeutisch akzeptablen Hilfsstoff.

9. Prasugrelzusammensetzung nach Anspruch 8, wobei die genannte Zusammensetzung Folgendes umfasst:
- die mikronisierte Prasugrelzusammensetzung nach Anspruch 7;
- ein Verdünnungsmittel;
- ein Sprengmittel; und
- wahlweise, ein Gleitmittel.

10. Prasugrelzusammensetzung nach einem der Ansprüche 8 oder 9, wobei die genannte Zusammensetzung kein Tensid enthält.

11. Prasugrelzusammensetzung nach einem der Ansprüche 8 bis 10, wobei die genannte Zusammensetzung Folgendes umfasst:
- 7-20 Gew.-% der mikronisierten Prasugrelzusammensetzung nach Anspruch 7;
- 60-80 Gew.-% eines Verdünnungsmittels ausgewählt aus der Gruppe bestehend aus Lactose, mikrokristalliner Cellulose und Kombinationen davon;
- 5-15 Gew.-% eines Sprengmittels ausgewählt aus der Gruppe bestehend aus Crospovidon, Croscarmellose-Natrium, niedrig substituierter Hydroxypropylcellulose, Natriumstärkeglykolat, ionischen Harzen wie Polacrilin-Kalium und Kombinationen davon, vorzugsweise ist es Croscarmellose-Natrium; und
- wahlweise, 2-4 Gew.-% eines Gleitmittels, vorzugsweise ist das Gleitmittel ein Saccharose-Fettsäureester.

12. Prasugrelzusammensetzung nach Anspruch 11, wobei die genannte Zusammensetzung Saccharose-Fettsäureester als Gleitmittel umfasst und der genannte Saccharose-Fettsäureester aus der Gruppe bestehend aus Saccharose-Monoestern von Laurinsäure, Palmitinsäure und Stearinsäure, und Kombinationen davon ausgewählt wird.

13. Prasugrelzusammensetzung nach einem der Ansprüche 8 bis 12, wobei die genannte Zusammensetzung in Form von beschichteten Tabletten ist.

14. Prasugrelzusammensetzung nach einem der Ansprüche 8 bis 13, für deren Verwendung in einer Methode zur Behandlung von Gerinnungsstörungen.

## Revendications

1. Procédé pour l'obtention d'une composition de base de prasugrel micronisée comprenant :
a) mélanger la base de prasugrel avec un excipient hydrophile insoluble dans l'eau, dans lequel l'excipient hydrophile insoluble dans l'eau est la crospovidone, optionnellement en combinaison avec d'autres excipients ; et
b) microniser la composition obtenue en a), dans lequel le rapport en poids de prasugrel par rapport à l'excipient hydrophile insoluble dans l'eau dans la composition obtenue en a) est 1 : 3.

2. Procédé selon la revendication 1, dans lequel la composition obtenue en a) consiste essentiellement en prasugrel et un excipient hydrophile insoluble dans l'eau.

3. Procédé selon l'une quelconque des revendications 1 à 2, dans lequel ladite base de prasugrel dans l'étape a) est la base de prasugrel sous forme cristalline.

4. Procédé selon l'une quelconque des revendications 1 à 3, dans lequel la composition obtenue en a) est micronisée dans l'étape b) jusqu'à une taille de particule moyenne inférieure à 90 µm, de préférence comprise entre 10 et 50 µm, lorsque la taille de particule moyenne est déterminée par spectrométrie par diffraction laser.

5. Procédé selon l'une quelconque des revendications 1 à 4, dans lequel l'étape de micronisation b) est **caractérisée par** une ou plusieurs, de préférence la totalité, des caractéristiques suivantes :
- le taux d'alimentation est de 50 rpm jusqu'à 300 rpm ;
- la vitesse de micronisation est de 18 jusqu'à 70 g/min ; et
- la pression de micronisation est de 1,5 jusqu'à 5,5 bar.

6. Procédé selon l'une quelconque des revendications 1 à 5, dans lequel ladite étape de micronisation est réalisée par broyage à sec.

7. Composition de prasugrel micronisée, dans laquelle ladite composition peut être obtenue par un procédé selon l'une quelconque des revendications 1 à 6.

8. Composition de prasugrel sous forme d'un comprimé dans laquelle ladite composition comprend :
i. la composition de prasugrel micronisée selon la revendication 7 ; et
ii. un excipient pharmaceutiquement acceptable.

9. Composition de prasugrel selon la revendication 8, dans laquelle ladite composition comprend :
- la composition de prasugrel micronisée selon la revendication 7 ;
- un diluant ;
- un délitant ; et
- optionnellement, un lubrifiant.

10. Composition de prasugrel selon l'une quelconque des revendications 8 ou 9, dans laquelle ladite composition ne contient pas de tensioactif.

11. Composition de prasugrel selon l'une quelconque des revendications 8 à 10, dans laquelle ladite composition comprend :
- 7-20 % en poids de la composition de prasugrel micronisée selon la revendication 7 ;
- 60-80 % en poids d'un diluant choisi dans le groupe constitué du lactose, de la cellulose microcristalline et des combinaisons de ceux-ci ;
- 5-15 % en poids d'un délitant choisi dans le groupe constitué de la crospovidone, de la croscarmellose de sodium, de l'hydroxypropyl-cellulose faiblement substituée, du glycolate d'amidon sodique, des résines ioniques telles que le sel de potassium de polacriline et des combinations de ceux-ci, de préférence il s'agit de la croscarmellose de sodium ; et
- optionnellement, 2-4 % en poids d'un lubrifiant, de préférence le lubrifiant est un ester d'acide gras de saccharose.

12. Composition de prasugrel selon la revendication 11, dans laquelle ladite composition comprend un ester d'acide gras de saccharose comme lubrifiant et ledit ester d'acide gras de saccharose est choisi dans le groupe constitué des monoesters de saccharose d'acide laurique, d'acide palmitique et d'acide stéarique, et des combinations de ceux-ci.

13. Composition de prasugrel selon l'une quelconque des revendications 8 à 12, dans laquelle ladite composition est sous forme de comprimés enrobés.

14. Composition de prasugrel selon l'une quelconque des revendications 8 à 13, pour son utilisation dans une méthode de traitement de troubles de la coagulation.
